# EUROPEAN PATENT APPLICATION

(11) **EP 1 205 216 A2**
(43) Date of publication of application: **15.05.2002**
(21) Application number: 01200568.2
(22) Date of filing: 16.02.2001
(51) Int. Cl.: A61N 5/10

(54) **Miniature x-ray source insulation structure**

(30) Priority: 09.11.2000 US 246722 P
(71) Applicant: Radi Medical Technologies AB, 754 50 Uppsala (SE)
(72) Inventor: Tirén, Jonas, 753 34 Uppsala (SE)
(74) Representative: Holmberg, Nils Anders Patrik

(57) **Abstract**

Miniature X-ray source comprising an insulation structure defining a cavity where an anode and a cathode are arranged, said cavity being evacuated, connecting means arranged to connect the anode and the cathode to a high voltage source in order to energise the X-ray source. The insulation structure includes a first layer and a second layer, the first layer facing the cavity and has a low gas permeability, and the second layer is arranged outside said first layer and has a high electrical break-through voltage. The electrical break-through voltage for the insulation structure is above a predetermined threshold value.

## Description

### Field of the invention

The present invention relates to an insulation structure for a miniature X-ray source and a miniature X-ray source according to the preambles of the independent claims.

### Background of the invention

In treating stenosis in coronary arteries, a restenosis occurs in 30-60% of the cases. It is known that a treatment with beta- or gamma- (X-ray) radiation will decrease the occurrence of restenosis substantially.

Another example of an application of the present invention is treatment of cancer tumors where it is desired to deliver radiation locally.

Methods to apply the radiation to the site of treatment are presently subject to intensive research. Generally, a hollow catheter is inserted into the body, typically via an artery, in such a way that its distal end is placed near the site of treatment. A source of radiation attached to the distal end of an elongated member is inserted into the hollow catheter, and is forwarded until the radiation source is disposed at a proper position for radiating the site of treatment. In the specific case of treating cardiac vessels, the catheter is placed near the cardiac vessel tree (this catheter often called a "guide catheter"). A very thin wire - called guide wire - is then used to probe further and reach the site where treatment shall be performed. The therapeutic device is moved along this wire, i.e. by threading the device onto the guide wire. It is obvious that the therapeutic device has to have a hole close to its distal end in order to do this.

Radiation treatment methods using radioactive pellets or balloons etc. as radiation source is known in the art. Since these methods have some drawbacks, such as the need for substantial efforts to control radiation in the environment outside the patient, the use of a miniature electrical X-ray source including a cold cathode has been proposed. Such a source may be switched on and off due to its electrical activation. An example of such an X-ray source is described in the US patent 5,854,822.

For the purpose of providing irradiation, such as X-ray, at a therapy location, e.g. radiation treatment of coronary arteries, the radiation source should preferably be capable of providing radiation at the same intensity in all circumferential directions, since the vessel can be compared to a tube having a circular cross-section. The radiation source is connected via a cable, preferably a coaxial cable, to an externally located high voltage source that supplies a high voltage in the order of 20-30 kV or more.

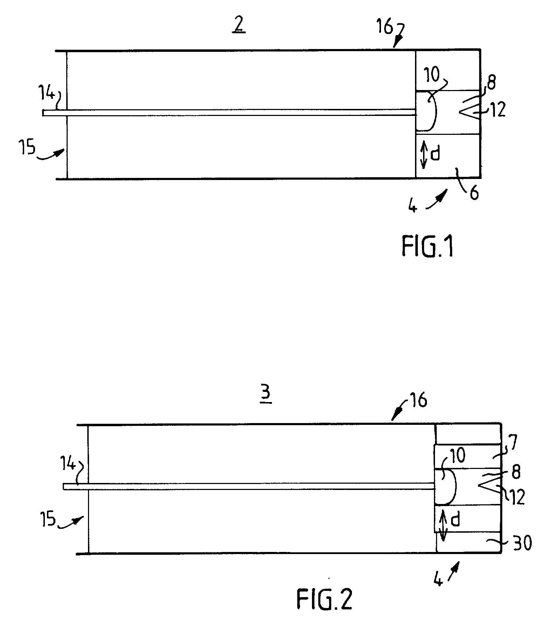

Figure 1 schematically illustrates a prior art device generally designated with reference numeral 2.

This prior art device comprises an X-ray source 4 located at the distal en of a suitable wire or catheter. The source comprises a support structure 6, essentially in the form of a tube. A through-going hole in said support structure provided with an anode 10 and a cathode 12 at opposite ends of the hole defines a vacuum cavity 8. The anode 10 is connected to a central conductor 14 of the coaxial cable 15, and the cathode 12 is connected via an outer conductor 16 to the external voltage source.

The outer diameter of the entire source should preferably be less than 1,8 mm, even more deisrable is a diameter less tha 1.3mm. The material thickness must not be too large, because the material will then to a larger degree absorb the radiation generated by the source. Also, the tube-like support structure 6 must be sufficiently gas impermeable to maintain a vacuum inside the cavity 8.

In summary the requirements to be met by a material used for the support structure are the following:
- Electrical break-through voltage higher than a preset level.
- Sufficiently gas impermeable to maintain vacuum inside the cavity.
- High mechanical stability.

One material known to the inventor that meets the requirement regarding the electrical break-through is pyrolytic boron nitride (pBN). This material has furthermore a low absorption of radiation in the energy range in question. Nevertheless, it has some drawbacks. It is relatively weak from a mechanical point of view, inter alia it is very anisotropic in terms of thermal expansion. Furthermore, it is expensive. Also, it is questionable if it is sufficiently gas impermeable, i.e. gas will probably diffuse through the material over time, rendering the shelf life unduly short, unless a coating is used as a diffusion barrier, which adds cost and complexity.

Other usable materials are alumina (polycrystalline Al₂O₃), sapphire (crystalline Al₂O₃), and quarts. The vacuum properties of these materials are sufficiently good, and they are substantially stronger than pyrolytic boron nitride. However, the electrical break-through voltage for these materials is only about 40 kV/mm. The distance d in figure 1 will result to be approximately 0,6-0,8 mm which results in that there is a danger that an electrical break-through occurs when using the above-mentioned voltages (20-30 kV) when energizing the X-ray source.

The object of the present invention is to arrange a structure for a miniature X-ray source having improved performance with regard to electrical break-through voltage, low gas permeability and mechanical strength.

### Summary of the invention

The above-mentioned object is achieved by an insulation structure and a miniature X-ray source according to the characterizing portions of the independent claims. Preferred embodiments are set forth in the dependent claims.

Thus, the present invention solves the above-mentioned problem by designing the support structure (or tube) using two different materials. A first usable material will have the capability to maintain a vacuum over extended periods of time, i.e. having a sufficiently low permeability to maintain a vacuum of 10⁻⁴ - 10⁻⁶ Torr over at least several months, preferably 1 year of more, most preferably 5 years or more. This material will be used for the inner part of the tube, forming the cavity in which the anode and cathode are disposed. The device uses field emission of electrons to operate and a too high pressure will result in a quenching of the electron current, and finally a breakdown and a generation of plasma. Furthermore, the authors have found that a better vacuum gives a more stable device with better control over its properties.

A second material will then be disposed on the first material and will serve as an insulating layer, for increasing the performance with regard to the electrical break-through voltage, such that the structure will be able to withstand the voltage applied.

### Short description of the appended drawings

Figure 1 illustrates schematically the general structure of a miniature X-ray source.

Figure 2 is a schematic illustration of a miniature X-ray source according to the present invention.

Figures 3a and 3b show a cross-sectional side view of the X-ray source and a top view of the bucket-like member, respectively, according to the present invention.

### Detailed description of preferred embodiments of the invention

In figure 2 is shown a device 3 comprising the insulation structure according to the present invention, wherein elements common to figure 1 are shown by the same reference numerals. The device comprises an X-ray source 4 located at the distal end of a suitable wire or catheter. The source comprises a support structure 7 (essentially a tube like member), made of alumina, sapphire or quartz, in which there is a hole forming a vacuum cavity 8. An anode 10 and a cathode 12 are provided in said cavity so as to seal the hole, thereby forming the cavity. The anode 10 is connected to a central conductor 14 of the coaxial cable 15, and the cathode 12 is connected via an outer conductor (shield) 16 to the external voltage source.

As is illustrated, the difference between the inventive structure and the device shown in figure 1 is that the support structure 7 has a smaller size than the support structure 6 of figure 1. The effective outer diameter (meaning the diameter of a circle that completely encloses the support structure in the event the support does not have a circular periphery) is in the range of 0.5 - 1.5mm. Furthermore, the support structure 7 is enclosed by a bucket-like member 30 in a material having a desired electrical break-through voltage that is higher than a predetermined threshold. Suitable material are polymers, such as poly-imide (KAPTON®), ultra high molecular weight polyethylene (UHMW PE), high density polyethylene,high density polyethylene, Teflon®, glass-fiber reinforced Teflon®. Pyrolytic boron nitride is also possible to use, but now as a insulator and not for any other off its good properties.

An X-ray source embodying the inventive insulation structure can be manufactured as follows.

A wafer of a suitable material is made either as a flat disc, in which holes for the vacuum cavities are made by e.g. laser drilling, or by making the wafer in a mold such that the holes are formed already during the manufacture of the wafer. If using alumina, the wafer may be precision machined before sintering

Then, anodes and cathodes are attached by suitable means, such as gluing soldering, bonding etc. in the holes so as to seal the cavity. The cavity must be evacuated, which can be achieved with methods known by the skilled person in the art such as by employing evacuation channels and getter materials, and will not be further discussed herein.

The wafer is then cut such that a desired shape of the individual elements is obtained. This can be square, hexagonal, octagonal, circular or some other desired shape. A material for the wafer can be selected from pyrolytic boron nitride (pBN), sapphire, quarts, alumina etc.

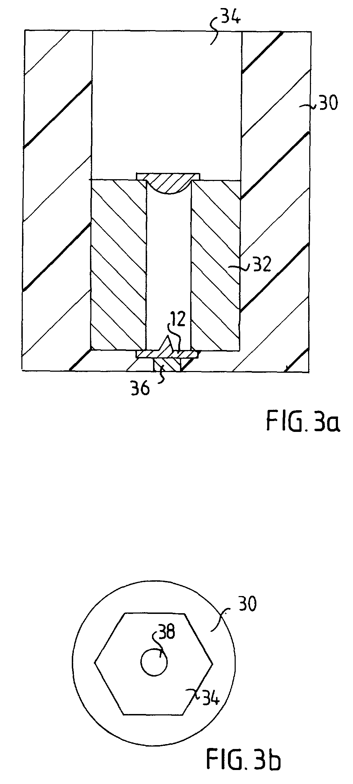

The individual elements are then to be embedded in a polymer. This is conveniently achieved by making the bucket-like member 30 by using an injection molding technique (see figure 3a). The bucket-like member 30, made of a suitable plastic (polymer) material is made such that it is able to receive the support structure 32 snugly, i.e. for a hexagonal support the receiving space 34 should also be hexagonal, see figure 3b, which is a view of the bucket-like member 30 from above. Conveniently the mold for the bucket is circular so as to produce a final X-ray source having a generally tubular shape, which is most suitable for insertion into blood vessels which have a generally tubular geometry. However, any shape can be made if desired. Suitable material are UHMW PE, Poly-imide, fluorocarbon polymers (e.g. Teflon®), or similar material having the required properties for achieving the purpose of the invention, namely a break-through voltage such that the combination of the ceramic support and the polymer can withstand an absolute voltage up to 30 kV or even more (20 kV + a safety margin). Naturally, even higher absolute voltages (e.g. up to 50 kV) are possible to achieve if suitable materials and dimensions are chosen, without departing from the scope of the present invention that is defined by the appended claims.

In order to make contact to the cathode 12 it is possible to deposit a thin layer (in the order of 0,1-50 µm thick) of metal on the exterior surface of the polymer bucket (not shown in figure 3a), after having ascertained that there is an exposed area of the cathode to which contact can be made. This exposed area can be obtained by making the bucket with a conductive piece 36 in its bottom, so as to contact the backside of the cathode when the support is placed in the bucket. Alternatively, the bucket can be made with a hole 38 in its bottom so as to expose the backside of the cathode through the hole, when mounted in the bucket. The deposition of metal will then automatically form the desired contact.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Miniature X-ray source insulation structure, said X-ray source comprising a high voltage anode (10) and a cathode (12) disposed in said insulation structure (7,30), **characterized in that** said insulation structure comprising a first layer (7,32) having a low gas permeability, and a second layer (30) having a high electrical break-through voltage, such that the insulation structure achieves predetermined requirements with regard to electrical break-through voltage and gas permeability.

2. Insulation structure according to claim 1, **characterized in that** said electrical break-through voltage requirement for the insulation structure is that the insulation structure withstands an absolute voltage of up to 30 kV.

3. Insulation structure according to claim 1, **characterized in that** said gas permeability requirement for the insulation structure is that the evacuated cavity maintains a vacuum of 10⁻⁴ torr for at least one year.

4. Insulation structure according to claim 1, **characterized in that** said second layer is disposed on said first layer.

5. Insulation structure according to claim 1, **characterized in that** the support structure constitutes the first layer of the insulation structure.

6. Insulation structure according to claim 5, **characterized in that** the support structure is made of a material selected from alumina, sapphire and quartz.

7. Insulation structure according to claim 1, **characterized in that** the second layer is made of a polymer.

8. Insulation structure according to claim 7, **characterized in that** the second layer is made of a material selected from poly-imide (KAPTON®), ultra high molecular weight polyethylene (UHMW PE), high density polyethylene,fluorocarbon polymers or glass-fiber reinforced fluorocarbon polymers (e.g. Teflon®).

9. Insulation structure according to claim 1, **characterized in that** the electrical break-through voltage for the material of the second layer is approximately >75 kV/mm.

10. Miniature X-ray source comprising an insulation structure defining a cavity (8) where an anode (10) and a cathode (12) are arranged, said cavity being evacuated, connecting means (14,16) arranged to connect the anode and the cathode to a high voltage source in order to energise the X-ray source, **characterized in that** the insulation structure includes a first layer (7,32) and a second layer (30), the first layer facing the cavity and has a low gas permeability, and the second layer is arranged outside said first layer and has a high electrical break-through voltage, wherein the electrical break-through voltage for the insulation structure is above a predetermined threshold value.

11. Miniature X-ray source according to claim 10, **characterized in that** said predetermined threshold value for the electrical break-through voltage is >30 kV.

12. Miniature X-ray source according to claim 10, **characterized in that** a support structure constitutes the first layer and a bucket-like member constitutes the second layer, said support structure is positioned in said bucket-like member,

13. Miniature X-ray source according to claim 10, **characterized in that** the support structure has a centrally located through-going hole, the anode and the cathode are attached in said hole such that they face each other and defines the cavity between them.

14. Miniature X-ray source according to claim 12, **characterized in that** said bucket-like member has a conducting portion connecting the cathode to the connecting means.

15. Miniature X-ray source according to claim 12, **characterized in that** the support structure is made of a material selected from alumina, sapphire and quartz.

16. Miniature X-ray source according to claim 12, **characterized in that** the bucket-like member is made of a material selected from poly-imide (KAPTON®), ultra high molecular weight polyethylene (UHMW PE), high density polyethylene,fluorocarbon polymers or glass-fiber reinforced fluorocarbon polymers (e.g. Teflon®).

17. Miniature X-ray source according to claim 10, **characterized in that** the outer diameter of the insulation structure is less than 1,5 mm.
